# EUROPÄISCHE PATENTSCHRIFT

(11) **EP 1 284 951 B1**
(45) Veröffentlichungstag und Bekanntmachung des Hinweises auf die Patenterteilung: **19.10.2005**
(21) Anmeldenummer: 01936297.9
(22) Anmeldetag: 28.04.2001
(51) Int. Cl.: C07C 47/11, C07C 47/225, C11B 9/00

(54) **3,3-DIMETHYLCYCLOHEXAN-DERIVATE**
3,3-DIMETHYLCYCLOHEXANE DERIVATIVES
DERIVES DE 3,3-DIMETHYLCYCLOHEXANE

(30) Priorität: 25.05.2000 DE 10026004
(43) Veröffentlichungstag der Anmeldung: 26.02.2003
(73) Patentinhaber: KAO CORPORATION, Chuo-Ku Tokyo 103-8210 (JP)
(72) Erfinder: MARKERT, Thomas, 40789 Monheim (DE); NEMITZ, Ralph, 41363 Jüchen (DE); PIERIK, Theo, Ten, NL-05916 LE Venlo (NL)
(74) Vertreter: Wolf, Matthias
(86) Internationale Anmeldenummer: PCT/EP2001/004820
(87) Internationale Veröffentlichungsnummer: WO 2001/090038

(56) Entgegenhaltungen:
- CH-A- 678 424
- DATABASE WPI Section Ch, Week 199210 Derwent Publications Ltd., London, GB; Class B07, AN 1992-076755 XP002177473 & JP 04 021649 A (TAKASAGO PERFUMERY CO LTD), 24. Januar 1992 (1992-01-24)

## Beschreibung

### Gebiet der Erfindung

Die vorliegende Erfindung betrifft neue 3,3-Dimethylcyclohexan-Derivate sowie deren Verwendung als Riechstoffe.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5000 kg Rosenblüten notwendig. Die Folgen sind eine sehr stark limitierte Weltjahresproduktion sowie ein hoher Preis. Es ist daher klar, dass die Riechstoffindustrie einen ständigen Bedarf an neuen Riechstoffen mit interessanten Duftnoten hat. Einerseits kann dadurch die Palette der natürlich verfügbaren Riechstoffe ergänzt werden, andererseits ist es dadurch möglich, die notwendigen Anpassungen an wechselnde modische Geschmacksrichtungen vornehmen zu können. Darüber hinaus wird es auf diese Weise möglich, den ständig steigenden Bedarf an Geruchsverbesserern für Produkte des täglichen Bedarfs wie Kosmetika und Reinigungsmittel decken zu können.

Im Übrigen besteht generell ein ständiger Bedarf an synthetischen Riechstoffen, die sich günstig und mit gleichbleibend hoher Qualität herstellen lassen und die originelle olfaktorische Eigenschaften haben. Insbesondere sollen sie angenehme, möglichst naturnahe und qualitativ neuartige Geruchsprofile von ausreichender Intensität besitzen und in der Lage sein, den Duft von kosmetischen und Verbrauchsgütern vorteilhaft zu beeinflussen. Mit anderen Worten: es besteht ein ständiger Bedarf an Verbindungen, die charakteristische neue Geruchsprofile bei gleichzeitig hoher Haftfestigkeit, Geruchsintensität und Strahlkraft aufweisen.

Aus CH-A-678 424 and JP-A-4 021 649 sind Cylohexan-Derivate und deren Verwendung als Rirchstoffe bekannt.

### Beschreibung der Erfindung

Es wurde gefunden, dass die Verbindungen der allgemeinen Formel (I) die oben genannten Forderungen in jeder Hinsicht ausgezeichnet erfüllen und in vorteilhafter Weise als Riechstoffe mit unterschiedlichen nuancierten Geruchsnoten mit guter Haftfestigkeit eingesetzt werden können.

Gegenstand der vorliegenden Erfindung sind zunächst 3,3-Dimethylcyclohexan-Derivate der allgemeinen Struktur (1) 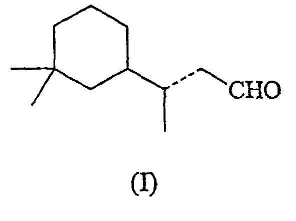 worin die gestrichelte Linie, die die zur Aldehydgruppe α- und β-ständigen Kohlenstoffatome verbindet, eine C-C-Einfachbindung oder eine cis- oder trans-konfigurierte C=C-Doppelbindung bedeutet.

In einer weiteren AusRhungsform betrifft die Erfindung die Verwendung von zunächst 3,3-Dimethylcyclohexan-Derivaten der oben näher bezeichneten allgemeinen Struktur (I) als Riechstoffe. Dabei können die Verbindungen sowohl einzeln als auch in Kombination untereinander eingesetzt werden.

Die erfindungsgemäßen Verbindungen (I) zeichnen sich durch eine Geruchscharakteristik aus, in der fruchtice und holzige Noten sowie Ionon-Aspekte dominieren. Sie weisen eine ausgezeichnete Stabilität in Rezepturen der Kosmetik und Gebrauchsparfümerie auf.

Die Herstellung der Verbindungen (I) kann nach bekannten Syntheseverfahren der organischen Chemie erfolgen

In Parfum-Kompositionen verstärken die Verbindungen (I) die Harmonie und die Ausstrahlung sowie die Natürlichkeit und auch die Haftung, wobei die Dosierung unter Berücksichtigung der übrigen Bestandteile der Komposition auf die jeweils angestrebte Duftnote abgestimmt wird.

Dass die Verbindungen (I) die oben genannten Duftnoten aufweisen, war nicht vorhersehbar und ist damit eine weitere Bestätigung für die allgemeine Erfahrung, dass die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluss der chemischen Struktur auf die Duftwahrnehmung hinreichend erforscht sind und somit also normalerweise nicht vorhergesehen werden kann, ob ein geänderter Aufbau bekannter Riechstoffe überhaupt zur Änderung der olfaktorischen Eigenschaften führt und ob diese Änderungen vom Fachmann positiv oder negativ beurteilt werden.

Die Verbindungen der Formel (I) eignen sich aufgrund ihres Geruchsprofiles insbesondere auch zur Modifizierung und Verstärkung bekannter Kompositionen. Hervorgehoben werden soll insbesondere ihre ausserordentliche Geruchsstärke, die ganz allgemein zur Veredelung der Komposition beiträgt.

Die Verbindungen der Formel (I) lassen sich mit zahlreichen bekannten Riechstoffingredienzien, beispielsweise anderen Riechstoffen natürlichen, synthetischen oder partial-synthetischen Ursprungs, ätherischen Ölen und Pflanzenextrakten kombinieren. Die Palette der natürlichen Riechstoffe kann dabei sowohl leicht-, als auch mittel- und schwerflüchtige Komponenten umfassen. Die Palette der synthetischen Riechstoffe kann Vertreter aus praktisch allen Stoffklassen umfassen.

Beispiele für geeignete Substanzen, mit denen die Verbindungen (I) kombiniert werden können sind insbesondere:
(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl, Myrrheöl, Olibanumöl
(b) Alkohole wie Farnesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],
(c) Aldehyde wie Citral, Helional^{R}, α-Hexylzimtaldehyd, Hydroxycitronellal, Lilial^{R} [p-tert.-Butyl-α-methyldihydrozimtaldehyd], Methylnonylacetaldehyd,
(d) Ketone wie Allylionon, α-Tonon, β-Ionon, Isoraldein, Methylionon,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat,
(f) Lactone wie gamma-Undecalacton, 1-Oxaspiro[4.4]nonan-2-on, sowie verschiedene weitere in der Parfümerie oft benutzte Komponenten wie Moschus- und Sandelholz-Riechstoffe, Indol,p-Menthan-8-thiol-3-on, Methyleugenol und Ambroxan.

Bemerkenswert ist ferner die Art und Weise, wie die Verbindungen der Struktur (I) die Geruchsnoten einer breiten Palette bekannter Kompositionen abrunden und harmonisieren, ohne jedoch in unangenehmer Weise zu dominieren. 3-(3,3-Dimethyl-cyclohex-1-yl)-butanal ist in dieser Hinsicht ganz besonders hervorzuheben.

Die einsetzbaren Anteile der erfindungsgemäßen Verbindungen (I) oder deren Gemische in Riechstoffkompositionen bewegen sich von etwa 1-70 Gew. %, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Pudern, Aerosolen, Zahnpasten, Mundwässern, Deodorantien als auch in der alkoholischen Parfümerie (z.B. Eau de Cologne, Eau de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,05 - 2 Gew. % - bezogen auf das gesamte Produkt - zugesetzt. Diese Werte sind jedoch keine beschränkenden Grenzwerte, da der erfahrenen Parfümeur auch mit noch geringeren Konzentrationen noch Effekte erzielen oder mit noch höheren Dosierungen neuartige Komplexe aufbauen kann.

### Beispiele

### Herstellvorschrift:

### Beispiel 1: Herstellung von 1,1-Diethoxy-1-(3,3-dimethyl-cyclohex-1-yl)-ethan

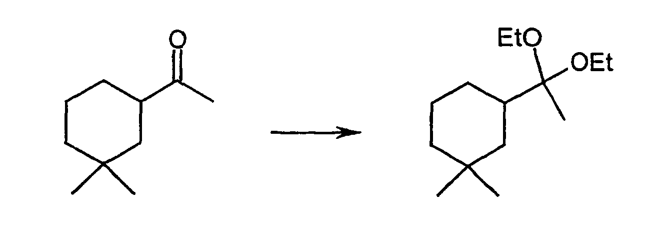

Ansatz:
   1) 154,0 g (1 mol) 1-(3,3-Dimethylcyclohex-1-yl)-ethanon
   2) 177,8 g (1,2 mol) Triethylorthoformiat
   3) 0,1874 g Kaliumhydrogensulfat
   4) 600 ml Ethanol
Apparatur: 2-1-Dreihalskolben, Rührer, Thermometer, Trockenrohr (Blaugel)
Ausführung: Die Komponenten 1), 2), 3), und 4 wurden nacheinander in den Reaktionskolben gegeben und 24 Stunden bei Raumtemperatur unter Feuchtigkeitsausschluß gerührt. Der Reaktionsverlauf wurde gachromatograohisch (GC) kontrolliert. Nach 24 Stunden hatten sich 76% Produkt gebildet. Nach weiteren 7 Stunden Rühren war kein weiterer Umsatz festzustellen. Die Reaktion wurde durch Zugabe von 2 ml Natriummethanolat-Lösung (30%ig in Methanol) neutralisiert. Am Rotationsverdampfer wurden im Wasserstrahlvakuum überschüssiges Ethanol und der gebildete Ameisensäureethylester abdestilliert. Das rohe Diethylketal des 1-(3,3-Dimethylcyclohex-1-yl)-ethanons wurde an einer 30 cm Füllkörper-Kolonne destilliert. Der Hauptlauf hatte eine GC-Reinheit von 78% und ging bei Siedetemperaturen von 70-72°C/0.1 mbar über.
Ausbeute: 51 % der Theorie
Analytik: Das ¹H-NMR-Spektrum (280 MHz, CDCl₃) zeigte 2 Singuletts für die geminalen Methylgruppen am Cyclohexanring bei 0,9 ppm (6H) sowie 2 Tripletts und 1 Singulett bei 1,2 ppm für die 3 restlichen Methylgruppen (9H). Die 2 Methylengruppen der Ethoxyreste ergaben 2 Quadrupletts bei 3,4 ppm, die sich überlagerten (4H) und die 4 Methylengruppen des Cyclohexanrings zeigten mehrere teilweise aufgespaltene und verbreiterte Signale über einen Bereich von 0,7 bis 2,0 ppm (8H). Das einzelne Proton der CH-Gruppe fiel auch in diesen Bereich und lag vermutlich unter den Methylgruppensignalen bei 1,2 ppm.
Geruchscharakteristik: Im Angeruch fruchtig, Johannisbeere, Damascone, erinnert an die Cognis-Riechstoffe Floramat® und Jasmacyclat®. Kein erkennbarer Nachgeruch nach 24 Stunden am Riechstreifen.

### Beispiel 2: Herstellung von 1,1,3-Triethoxy-3-(3,3-dimethylcyclohex-1-yl)-butan

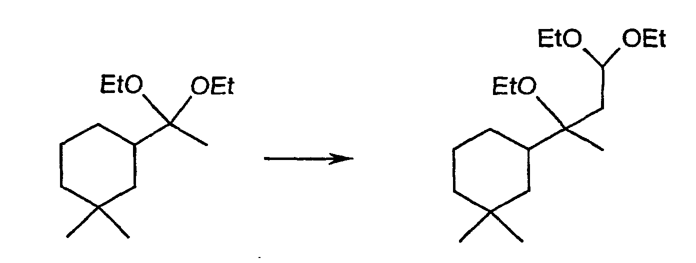

Ansatz:
   1) 104,3 g (0,46 mol) 1,1-Diethoxy-1-(3,3-dimethyl-cyclohex-1-yl)-ethan, hergestellt nach Beispiel1
   2) 36,6 ml Zinkchloridlösung (10%ig in Essigester)
   3) 48,0 ml (0,5 mol) Ethylvinylether (Fa. Acros)
Apparatur: 500-ml-Dreihalskolben mit Rührer, Thermometer, Rückflußkühler und Tropftrichter.
Ausführung: Die Komponenten 1) und 2) wurden nacheinander in den Reaktionskolben eingewogen und unter Rühren auf 50°C erwärmt. Die Komponente 3) wurde innerhalb 0,5 Stunden unter Rühren kontinuierlich zudosiert. Dabei stieg die Temperatur der Mischung auf 53°C an. Es wurde noch 3 Stunden bei 48° C gerührt. Bei der Umsatzkontrolle war das Edukt vollständig abreagiert, die Hauptkomponente hatte 49% Anteil.
Aufarbeitung: Der Ansatz wurde in einen Scheidetrichter überführt und mit Wasser und Natriumhydrogencarbonatlösung neutral gewaschen. Die organische Phase wurde über Natriumsulfat getrocknet und am Rotationsverdampfer eingeengt.

Es wurden 125 g Rohprodukt an einer 30 cm Füllkörperkolonne destilliert. Im Hauptlauf wurden 71,2 g 1,1,3-Triethoxy-3-(3,3-dimethylcyclohex-1-yl)-butan erhalten (Siedepunkt 96-108°C/0.08 mbar, GC-Reinheit 97,8%).
Ausbeute: 56% der Theorie
Analytik: Das ¹H-NMR-Spektrum (in CDCl₃, 280 MHz) zeigte die 2 geminalen Methylgruppen als Pseudosinguletts bei 0,9 ppm , 1 Methylgruppe als 2 Singuletts bei 1,1 ppm und die 3 Methylgruppen der Ethoxyreste als Tripletts bei 1,1 und 1,2 ppm. Die 4 Methylengruppen des Cyclohexanrings erschienen im Bereich zwischen 0,8 bis 1,8 ppm als stark verbreiterte Signale. Die 3 Methylengruppen aus Ethoxyresten gaben Quadrupletts bei 3,3; 3,5 und 3,6 ppm. Die Methylengruppe der Seitenkette zeigte 2 überlagerte Dubletts bei 1,8 ppm. Das Acetalproton ergab 2 Tripletts bei 4,7 ppm.
Geruchscharakteristik: Im Angeruch fruchtig, Rum-Aroma, alkoholisch, Grappa-Note. Nach 24 Stunden am Riechstreifen war kein Geruch mehr wahrnehmbar.

### Beispiel 3: Herstellung von 3-(3,3-Dimethyl-cyclohex-1-yl)-but-2-en-1-al

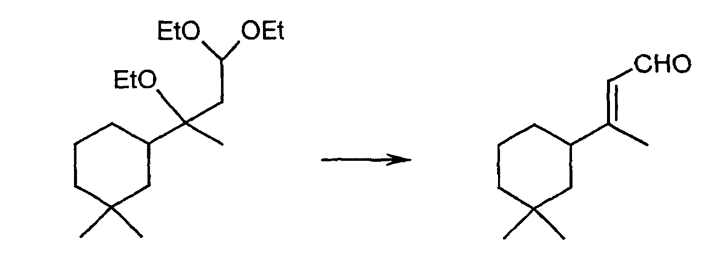

Ansatz:
   1) 65,2 g (0,22 mol) 1,1,3-Triethoxy-3-(3,3-dimethylcyclohex-1-yl)-butan (hergestellt nach Beispiel.2)
   2) 287,9 g Eisessig (Riedel de Haen)
   3) 30,1 g Natriumacetat
   4) 20,2 g Wasser
Apparatur: 1-1-Rührapparatur mit Thermometer, Rückflußkühler.
Ausführung: Die Komponenten 1), 2), 3) und 4) wurden vorgelegt und 5 Stunden bei 90°C gerührt. Das Reaktionsgemisch wurde über Nacht auf Raumtemperatur abgekühlen gelassen. Das GC der organischen Phase zeigte vollständigen Umsatz an.
Aufarbeitung: Das Gemisch wurde auf Eis gegossen, neutralisiert, die organische Phase abgetrennt und die Wasserphase mit Ether extrahiert. Die organischen Phasen wurden über Natriumsulfat getrocknet und am Rotationsverdampfer eingeengt.
29,2 g Rückstand wurde an einer 30 cm Füllkörperkolonne destilliert. Es wurden 20,5 g Hauptlauf isoliert, der bei Siedetemperaturen von 70 - 72°C/0.06 mbar überging (GC-Reinheit 97%).
Analytik: Das ¹H-NMR-Spektrum (400 MHz, in CDCl₃) war sehr komplex und wird wie folgt interpretiert: 1 Singulett bei 0,9 ppm steht für die beiden geminalen Methylgruppen am Cyclohexanring. Da dieses Signal durch den nicht zuzuordnenden Signalwald zwischen 0,8 und 1,8 ppm unterlegt ist, ist nur eine Integration des gesamten Bereichs bis 2,3 ppm möglich, der etwas mehr als den 18 erwarteten Protonen entspricht. Auffallend sind 2 ähnlich aussehende schwach aufgespaltene Singuletts bei 1,9 und 2,2 ppm , die Integration weist auf 3 Protonen, wovon 15% auf das Signal bei 1,9 ppm und 85% auf das Signal bei 2,2 ppm entfallen. Offensichtlich liegt eine Hauptkomponente und eine Nebenkomponente im Gemisch vor. Die beiden Signale werden der Methylgruppe der Seitenkette zugeordnet. Die Aufspaltung scheint mit der Aufspaltung des olefinischen Protons, Hauptkomponente bei 5,9 ppm, zu einem Pseudotriplett vom Dublett zu korrelieren. Die Dublettaufspaltung des olefinischen Protons zeigt die gleiche Kopplungskonstante wie das Aldehydproton bei 10,0 ppm. Eine Nebenkomponente ist mit schwachem Signal für das olefinische Proton bei 5,8 ppm und Aldehydproton bei 10,05 ppm zu erkennen.
Geruchscharakteristik: Im Angeruch grün, trocken, staubig, fruchtig, Cassis, Ionon, Holz. Im Nachgeruch nach 24 Stunden am Riechstreifen: frisch, fruchtig, holzig

### Beispiel 4: Herstellung von 3-(3,3-Dimethyl-cyclohex-1-yl)-butanal

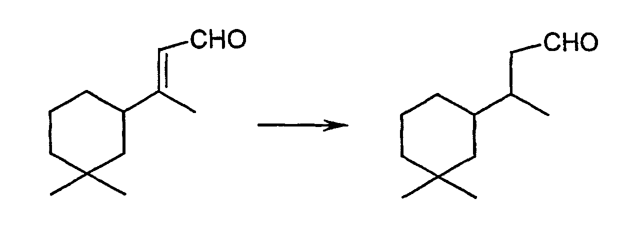

Ansatz:
   1) 18,0 g (0,1 mol) 3-(3,3-Dimethyl-cyclohex-1-yl)-but-2-en-1-al, hergestellt nach Beispiel 3
   2) 0,1 g RCh-Katalysator 50/8 (Hoechst) 5% Pd/C (RCh steht abkürzend für "Ruhrchemie")
   3) 100 ml Cyclohexan
Apparatur: Hochdruck-Stahlautoklav
Ausführung: Die Komponenten 1), 2) und 3) wurden nacheinander in den Autoklaveneinsatz eingewogen. Anschließend wurde unter Rühren bei 70°C und 20 bar Wasserstoffüberdruck 7 Stunden lang hydriert.
Aufarbeitung: Nach Abkühlen und Entspannen wurde der Katalysator abfiltriert und das Reaktionsgemisch am Rotationsverdampfer eingeengt. 14,5 g Rückstand wurden an einer 30 cm Füllkörperkolonne destilliert. Bei Siedetemperaturen von 53-55°C/0.06 mbar wurden Produktfraktionen erhalten, deren Gehalt an 3-(3,3-Dimethyl-cyclohex-1-yl)-butanal mehr als 90% entsprach (GC-Messung).
Analytik: Das ¹H-NMR-Spektrum (280 MHz, in CDCl₃) zeigte 2 Singuletts und 1 Dublett der 3 Methylgruppen (9H) bei 0,9 ppm. Im Bereich zwischen 1,2 und 2,0 ppm erschienen die 4 CH₂-Gruppen des Cyclohexanrings, während die Signale für die beiden Einzelprotonen vermutlich im Bereich zwischen 0,8 und 1,2 ppm lagen und teilweise von den Signalen für die Methylgruppen verdeckt waren. Die der Aldehydgruppe benachbarte CH₂-Gruppe zeigte zwei entartete ddd- oder ddt-Signale bei 2,2 und 2,5 ppm, die gleiche Kopplungskonstanten aufwiesen wie das dd-Aldehydprotonensignal bei 9,8 ppm.
Geruchscharakteristik: Im Angeruch grün, frisch, trocken, aldehydisch, blumig, strahlend und im Nachgeruch nach 24 Stunden am Riechstreifen strahlend, trocken, holzig, fruchtig.

## Patentansprüche

1. 3,3-Dimethyleyelohexan-Derivate der allgemeinen Struktur (I) 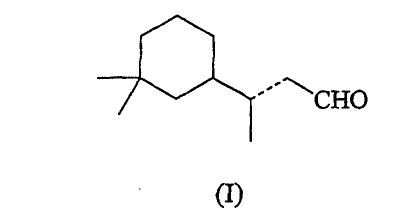 worin die gestrichelte Linie, die die zur Aldehydgruppe α- und β-ständigen Kohlenstoffatome verbindet, eine C-C-Einfachbindung oder eine cis- oder trans-konfigurierte C=C-Doppelbindung bedeutet.

2. Verwendung von 3,3-Dimethylcyclohexan-Derivaten der allgemeinen Struktur (I) 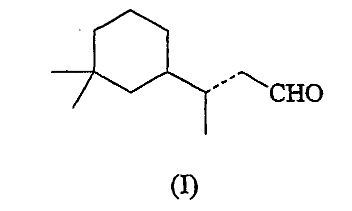 worin die gestrichelte Linie, die die zur Aldehydgruppe α- und β-ständigen Kohlenstoffatome verbindet, eine C-C-Einfachbindung oder eine cis- oder trans-konfigurierte C=C-Doppelbindung bedeutet, als Riechstoffe.

3. Riechstoff-Kompositionen mit einem Gehalt an einem oder 3,3-Dimethylcyclohexan-Derivaten (I) gemäß Anspruch 1, wobei die Verbindungen (I) in den Kompositionen in einer Menge von 1 bis 70 Gew.-% - bezogen auf die gesamte Komposition - enthalten sind.

## Claims

1. 3,3-Dimethylcyclohexane derivatives of the general structure (I) 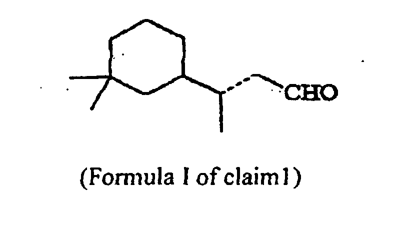 wherein the dashed line, which joins the α-position and β-position carbon atoms relative to the aldehyde group, denotes a C-C single bond or a cis-configured or trans-configured C=C double bond.

2. Use of 3,3-dimethylcyclohexane derivatives of the general structure (I) 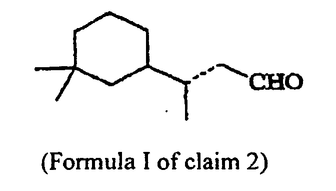 wherein the dashed line, which joins the α-position and β-position carbon atoms relative to the aldehyde group, denotes a C-C single bond or a cis-configured or trans-configured C=C double bond, as perfumes.

3. Perfume compositions containing one or 3,3-dimethylcyclohexane derivatives (I) according to claim 1, wherein the compounds (I) are present in the compositions in a quantity of 1 to 70 wt.% - based on the total composition.

## Revendications

1. Dérivés de 3,3-diméthylcyclohexane ayant la structure générale (I) 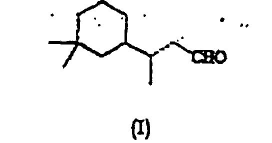 dans laquelle la ligne en pointillés, qui relie les atomes de carbone α et β-permanents pour le groupe aldéhyde, signifie une liaison simple C-C ou une double liaison C=C configurée cis ou trans.

2. Utilisation de dérivés de 3,3-diméthylcyclohexane ayant la structure générale (I) 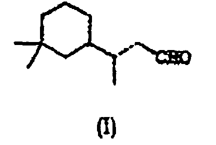 dans laquelle la ligne en pointillés, qui relie les atomes de carbone α et β-permanents pour le groupe aldéhyde, signifie une liaison simple C-C ou une double liaison C=C configurée cis ou trans, en tant que matières odorantes.

3. Compositions de matières odorantes ayant une teneur en un ou des dérivés de 3,3-diméthylcyclohexane selon la revendication 1, les composés (I) étant contenus dans les compositions en quantité de 1 à 70 % en poids - rapportée à la composition totale.
